# EUROPEAN PATENT APPLICATION

(11) **EP 3 502 691 A1**
(43) Date of publication of application: **26.06.2019**
(21) Application number: 17208949.2
(22) Date of filing: 20.12.2017
(51) Int. Cl.: G01N 33/50, G01N 33/68

(54) **PROADRENOMEDULLIN AS INDICATOR FOR RENAL REPLACEMENT THERAPY IN CRITICALLY ILL PATIENTS**

(71) Applicant: B.R.A.H.M.S GmbH, 16761 Hennigsdorf (DE)
(72) Inventor: WILSON, Darius, 79539 Lörrach (DE)
(74) Representative: Vossius & Partner Patentanwälte Rechtsanwälte mbB

(57) **Abstract**

The present invention relates to a method for assessing whether a critically ill subject is in need of a renal replacement therapy, wherein the method comprises determining in a sample obtained from said subject the level of proadrenomedullin (proADM) or a fragment thereof, preferably the fragment is MR-proADM, wherein said level of proADM or the fragment thereof is indicative of a need of said subject to receive a renal replacement therapy.

SEQUENCE LISTING

<110> B.R.A.H.M.S GmbH

<120> Proadrenomedullin as indicator for renal replacement therapy in
critically ill patients

<130> AA3060 EP BLN

<160> 2

<170> BiSSAP 1.3

<210> 1
<211> 185
<212> PRT
<213> Homo sapiens

<220>
<223> amino acid sequence of pre-pro-ADM

<400> 1
Met Lys Leu Val Ser Val Ala Leu Met Tyr Leu Gly Ser Leu Ala Phe
1               5                   10                  15
Leu Gly Ala Asp Thr Ala Arg Leu Asp Val Ala Ser Glu Phe Arg Lys
            20                  25                  30
Lys Trp Asn Lys Trp Ala Leu Ser Arg Gly Lys Arg Glu Leu Arg Met
        35                  40                  45
Ser Ser Ser Tyr Pro Thr Gly Leu Ala Asp Val Lys Ala Gly Pro Ala
    50                  55                  60
Gln Thr Leu Ile Arg Pro Gln Asp Met Lys Gly Ala Ser Arg Ser Pro
65                  70                  75                  80
Glu Asp Ser Ser Pro Asp Ala Ala Arg Ile Arg Val Lys Arg Tyr Arg
                85                  90                  95
Gln Ser Met Asn Asn Phe Gln Gly Leu Arg Ser Phe Gly Cys Arg Phe
            100                 105                 110
Gly Thr Cys Thr Val Gln Lys Leu Ala His Gln Ile Tyr Gln Phe Thr
        115                 120                 125
Asp Lys Asp Lys Asp Asn Val Ala Pro Arg Ser Lys Ile Ser Pro Gln
    130                 135                 140
Gly Tyr Gly Arg Arg Arg Arg Arg Ser Leu Pro Glu Ala Gly Pro Gly
145                 150                 155                 160
Arg Thr Leu Val Ser Ser Lys Pro Gln Ala His Gly Ala Pro Ala Pro
                165                 170                 175
Pro Ser Gly Ser Ala Pro His Phe Leu
            180                 185

<210> 2
<211> 48
<212> PRT
<213> Homo sapiens

<220>
<223> amino acid sequence of MR-pro-ADM (amino acid residues 45-92 of
pre-pro-ADM)

<400> 2
Glu Leu Arg Met Ser Ser Ser Tyr Pro Thr Gly Leu Ala Asp Val Lys
1               5                   10                  15
Ala Gly Pro Ala Gln Thr Leu Ile Arg Pro Gln Asp Met Lys Gly Ala
            20                  25                  30
Ser Arg Ser Pro Glu Asp Ser Ser Pro Asp Ala Ala Arg Ile Arg Val
        35                  40                  45

## Description

The present invention relates to a method for assessing whether a critically ill subject is in need of a renal replacement therapy. The assessment is based on the level of proadrenomedullin (proADM) or a fragment thereof such as MR-proADM in a sample of the subject.

### Background of the invention

Intensive care specialists are daily challenged to provide best quality of care to critically ill patients that are at high risk to die most frequently from multiple organ dysfunction syndrome (MODS) (Mayr, V. D., M. W. Dünser, et al. (2006), Crit Care 10(6): R154). Kidney dysfunction constitutes a substantial component in MODS and renal support becomes more and more routinely applied in intensive care units (ICUs). Sepsis is the predominant cause of acute kidney failure in critically ill patients (Bagshaw, S. M., S. Uchino, et al. (2007), Clin J Am Soc Nephrol 2(3): 431-439). About half of all patients with septic shock are complicated by kidney failure which is associated with increased mortality of more than 60 % and usually necessitates renal replacement therapy (RRT) (Schrier, R. W. and W. Wang (2004), N Engl J Med 351(2): 159-169; Uchino, S., J. A. Kellum, et al. (2005), JAMA 294(7): 813-818). Acute renal failure is a clinical syndrome that comprises a broad spectrum of declined renal function. Several classification systems (RIFLE, AKIN and KDIGO) exist staging severity of renal failure based on serum creatinine, glomerular filtration rate and urine output (Thomas, M. E., C. Blaine, et al. (2015), Kidney Int 87(1): 62-73). Renal replacement therapy is commonly indicated when renal dysfunction affects calcium balance or acid-base equilibrium, causes pulmonary edema, intoxications and complications such as pericarditis, encephalopathy or bleeding (Pannu, N. and R. N. Gibney (2005), Ther Clin Risk Manag 1(2): 141-150; Fleming, G. M. (2011), Organogenesis 7(1): 2-12; Ricci, Z., S. Romagnoli, et al. (2016), F1000Res 5). Clear guidelines on timing of RRT are missing although there is evidence that starting RRT early improves patient outcome compared with delayed or late start after indication for therapy (Pannu 2005, loc. cit.; Palevsky, P. M. (2008), Crit Care Med 36(4 Suppl): S224-228; Ricci 2016, loc. cit.). Modalities for RRT include intermittent (3-12 hours), continuous (24 hours) as well as hybrid techniques (Pannu 2005, loc. cit.; Fleming 2011, loc. cit.; Ricci 2016, loc. cit.). Continuous RRT is most commonly administered and recommended as it is applicable for hemodynamically unstable patients (Bagshaw, S. M., R. Bellomo, et al. (2010), Can J Anaesth 57(11): 999-1013; Fleming 2011, loc. cit.). All modes of RRT use semi-permeable membranes that apply mechanism of diffusion (hemodialysis), convection (hemofiltration) or a combination of both (hemodiafiltration) (Pannu 2005, loc. cit.; Ricci 2016, loc. cit.). Renal replacement therapy may comprise a mix of these techniques depending on the patients' clinical picture with continuous mode usually applied in the acute phase and intermittent mode following after stabilization. Decision making is highly complex with critical factors including patient conditions, benefits and side effects of RRT mode, compatibility with other therapeutic interventions, financial and organizational aspects as well as level of expertise (Pannu 2005, loc. cit.; Palevsky 2008, loc. cit.; Ricci 2016, loc. cit.).

Using current tools available in the Emergency Department (ED) and Intensive Care Unit (ICU), it can be difficult to accurately determine which patients are likely to develop renal complications in the short and mid-term and therefore are in need of renal replacement therapy (RRT). Numerous treatments are available once renal complications or renal dysfunction/failure become apparent to the treating physician. However, the key lies in the earlier initiation of treatment or to withhold or terminate a renal replacement therapy.
Both the early initiation of RRT and the rule out of RRT requirement are of significant clinical interest.

Therefore, the technical problem underlying the invention is the provision of means and methods to provide a simple and improved therapy of critically ill patients.

The technical problem is solved by provision of the embodiments provided herein below and as characterized in the appended claims.

### Description of the invention

The present invention is based on the surprising finding that the level of proadrenomedullin (proADM) or a fragment thereof, particularly midregional proadrenomedullin (MR-proADM), in a sample of a critically ill subject is indicative of the subject's need to receive a therapy directed at preventing and/or treating renal dysfunction. In the appended examples, the results of a clinical study are documented. This clinical study demonstrates that among all biomarkers tested, pro-adrenomedullin, reflected by MR-pro-ADM, has an unexpectedly strong statistical relationship with the critically ill subject's need (particularly subjects with sepsis or septic shock) to receive renal replacement therapy (RRT). Moreover, the study results indicate that the early initiation of RRT plays a role in decreasing the overall mortality rate. The present invention has, inter alia, the following advantages over conventional methods: the inventive method is fast, easy to perform and precise for determining the need to receive renal replacement therapy of a critically ill subject.

An object of the invention is to provide an in vitro method for risk assessment, risk stratification and/or therapy control in a subject and/or a patient, which provides reliable information especially to the medical practitioner in the Emergency Department (ED) or Intensive Care Unit (ICU). The present invention, thus, relates to a method for the therapy control, therapy guidance, monitoring, risk assessment, and/or risk stratification of a critically ill subject, wherein said method comprises determining the level of proadrenomedullin (proADM) or a fragment thereof, preferably MR-proADM, in a sample of said subject, wherein said level of proADM or a fragment thereof, preferably MR-proADM, is indicative of a need of said subject to receive a therapy, particularly renal replacement therapy.

The term "therapy control" in the context of the present invention refers to the monitoring and/or adjustment of a therapeutic treatment (here: particularly the renal replacement therapy) of said patient. "Monitoring" relates to keeping track of an already diagnosed disease, disorder, complication or risk, e.g. to analyze the progression of the disease or the influence of a particular treatment on the progression of disease or disorder. In the present invention, the terms "risk assessment" and "risk stratification" relate to the grouping of subjects into different risk groups according to their further prognosis. Risk assessment also relates to stratification for applying preventive and/or therapeutic measures.

More in particular, the present invention pertains to a method for assessing whether a critically ill subject is in need of a renal replacement therapy, wherein the method comprises determining in a sample obtained from said subject the level of proadrenomedullin (proADM) or a fragment thereof, preferably MR-proADM, wherein said level of proADM or the fragment thereof is indicative of a need of said subject to receive a renal replacement therapy. Hence, the method of the present invention can provide an assessment of
(i) whether a subject that does currently not receive a RRT should receive an RRT or not, and
(ii) whether a subject that currently receives a RRT should continue to receive said RRT or whether said RRT should be discontinued.

Consequently, in one aspect the present invention also relates to a method for assessing whether a critically ill subject is not in need of a renal replacement therapy, wherein the method comprises determining in a sample obtained from said subject the level of proadrenomedullin (proADM) or a fragment thereof, preferably MR-proADM, wherein said level of proADM or the fragment thereof is indicative of no need of said subject to receive a renal replacement therapy.

Typically, said level of proADM or the fragment thereof is compared to a reference level, and a need of said subject to receive said therapy is identified based on the comparison of the level of proADM or the fragment thereof with the reference level. Such a reference level can e.g. be a predetermined level that has been determined based on a population of healthy subjects who are not in need of renal replacement therapy or a population of critically ill subjects who are not in need of renal replacement therapy.
Herein, a level of proADM or said fragment thereof, preferably MR-proADM, above said reference level in the sample is indicative for a need of said therapy, particularly renal replacement therapy.

The reference level (i.e. the threshold) can be selected based on the individual requirements regarding specificity and/or selectivity of the assay as will be discussed herein below in more detail. Typically said reference level for proADM or a fragment thereof, preferably MR-proADM, is selected in the range of from 1 nmol/L to 12 nmol/L, preferably in the range of from 2 nmol/L to 11 nmol/L, more preferably in the range of from 2.5 nmol/L to 11 nmol/L, even more preferably between in the range of from 9 nmol/L to 11 nmol/L. Preferably said reference level is selected from the group consisting of 2.7 nmol/L, 2.8 nmol/L, 9.5 nmol/L and 10.9 nmol/L, more preferably herein the reference level is 2.7 nmol/L.

In one aspect of the method according to the invention, the critically ill subject is (already) receiving a renal replacement therapy, wherein said level of proadrenomedullin (proADM) or the fragment thereof is indicative to terminate the conducted renal replacement therapy. In particular, said RRT can be terminated when said level of proadrenomedullin (proADM) is below or equal to a reference level of 2.0 nmol/L, preferably equal or below 2.5 nmol/L, more preferably equal or below 2.7 nmol/L.

Hence, the method may in such cases comprise determining in a sample obtained from said subject the level of proadrenomedullin (proADM) or a fragment thereof, preferably MR-proADM, wherein said level of proADM or the fragment thereof is indicative of no need of said subject to receive a renal replacement therapy. Accordingly a renal replacement therapy can be withheld in patients based on the level of proadrenomedullin. Furthermore an already conducted renal replacement therapy can be stopped based on the level of proadrenomedullin, preferably if the level of proadrenomedullin is equal or below 2.0 nmol/L or 2.5 nmol/L or 2.7 nmol/L.

Conversely, if said level of proadrenomedullin (proADM) is above a reference level selected in the range of 2.7 nmol/L - 9.0 nmol/L, preferably above 2.7 nmol/L, more preferably above 9.0 nmol/L, more preferably above 10 nmol/L, most preferably above 10.9 nmol/L or 11 nmol/L, said subject is not in need of a RRT and/or the RRT can be stopped as the case may be.

The cut-off values of the protein level of proadrenomedullin or of other biomarkers, disclosed herein refer preferably to measurements in a sample obtained from a patient by means of the Thermo Scientific BRAHMS KRYPTOR assay. Accordingly, the values disclosed herein may vary to some extent depending on the detection/measurement method employed or vary due to different automated systems, and the specific values disclosed herein are intended to also read on the corresponding values determined by other methods.

The sensitivity and specificity of a diagnostic or prognostic method as the method of the present invention depends on more than just the analytical quality of the test, it also depends on the definition of what constitutes an abnormal or normal result. The distribution of levels of proADM or a fragment thereof, preferably levels of MR-proADM, for subjects with and without the need for RRT might overlap. Under such conditions, a test does not absolutely distinguish normal from subjects with a need for RRT with 100% accuracy. In other words, a balance between the inclusion of false negative and false positive results has to be found. The skilled person is aware of the fact that the condition per se of a subject or at least one further maker and/or parameter of the subject can assist in the interpretation of the data and that this further information allows a more reliable prognosis in the areas of overlap.

As used herein, a "critically ill patient" or "critically ill subject" means that the subject or patient is in a life threatening situation.
A critically ill subject (also referred to as a critically ill patient) herein is typically a subject undergoing intensive care or a subject that is a patient in an intensive care unit (ICU) or an Emergency Department (ED), particularly in an intensive care unit (ICU). In particular aspect, the critically ill patient suffers from sepsis or septic shock or is suspected to suffer from sepsis or septic shock.
The patient described herein who has been diagnosed as being "critically ill" can be diagnosed as an intensive care unit (ICU) patient, a patient who requires constant and/or intense observation of his/her health state, a patient diagnosed with sepsis, severe sepsis or septic shock, a patient diagnosed with an infectious disease and one or more existing organ failure(s), a pre- or post-surgical patient, a posttraumatic patient, a trauma patient, such as an accident patient, a burn patient, a patient with one or more open lesions. The subject described herein can be at the emergency department or intensive care unit, or in other point of care settings, such as in an emergency transporter, such as an ambulance, or at a general practitioner, who is confronted with a patient with said symptoms. Patients that are suspected to suffer from SIRS are not necessarily considered to be critically ill.

The term "ICU-patient" relates, without limitation, to a patient who has been admitted to an intensive care unit. An intensive care unit can also be termed an intensive therapy unit or intensive treatment unit (ITU) or critical care unit (CCU), is a special department of a hospital or health care facility that provides intensive treatment medicine. ICU-patients usually suffer from severe and life-threatening illnesses and injuries, which require constant, close monitoring and support from specialist equipment and medications in order to ensure normal bodily functions. Common conditions that are treated within ICUs include, without limitation, acute or adult respiratory distress syndrome (ARDS), trauma, organ failure and sepsis.

In certain aspects of the present invention, the critically ill patient has a SOFA score of 2 or more.

"Sepsis" in the context of the invention refers to a systemic response to infection. Sepsis may be characterized as clinical syndrome defined by the presence of both infection and a systemic inflammatory response (Levy MM et al. 2001 SCCM/ESICM/ACCP/ATS/SIS International Sepsis Definitions Conference. Crit Care Med. 2003 Apr;31(4):1250-6). The term "sepsis" used herein includes, but is not limited to, sepsis, severe sepsis, and septic shock. Severe sepsis in this context means sepsis associated with organ dysfunction, hypoperfusion abnormality, or sepsis-induced hypotension. Hypoperfusion abnormalities include lactic acidosis, oliguria and acute alteration of mental status. Sepsis-induced hypotension is defined by the presence of a systolic blood pressure of less than about 90 mm Hg or its reduction by about 40 mm Hg or more from baseline in the absence of other causes for hypotension (e.g. cardiogenic shock). Septic shock is defined as severe sepsis with sepsis-induced hypotension persisting despite adequate fluid resuscitation, along with the presence of hypoperfusion abnormalities or organ dysfunction (Bone et al., CHEST 101(6): 1644-55, 1992).
The term "sepsis" used herein relates to all possible stages in the development of sepsis.

As used herein, "infection" within the scope of the invention means a pathological process caused by the invasion of normally sterile tissue or fluid by pathogenic or potentially pathogenic microorganisms and relates to infection(s) by bacteria, viruses, fungi, and/or parasites. Accordingly, the infection can be a bacterial infection, viral infection, and/or fungal infection. The infection can be a local or systemic infection. Further, the subject suffering from an infection can suffer from more than one source(s) of infection simultaneously. For example, the subject suffering from an infection can suffer from a bacterial infection and viral infection; from a viral infection and fungal infection; from a bacterial and fungal infection, and from a bacterial infection, fungal infection and viral infection.

Herein said sample is a sample of a bodily fluid, preferably blood, plasma, serum, more preferably plasma or serum. The sample can for example be taken upon admission to the ICU so that the method assesses the subject's need to receive renal replacement therapy in the next one to seven days. However, typically once the subject's need to receive renal replacement therapy is determined according to the methods of the present invention, the subject will receive said renal replacement therapy as soon as possible.

For the purposes of the present invention the "subject" (or "patient") may be a mammal. In the context of the present invention, the term "subject" includes both humans and other mammals. Thus, the herein provided methods are applicable to both human and animal subjects, i.e. the method can be used for medical and veterinary purposes. Accordingly, said subject may be an animal such as a mouse, rat, hamster, rabbit, guinea pig, ferret, cat, dog, sheep, bovine species, horse, camel, or primate. Most preferably the subject is human.

Adrenomedullin (ADM) is encoded as a precursor peptide comprising 185 amino acids ("preproadrenomedullin" or "pre-proADM"), herein given in SEQ ID NO: 1. ADM comprises the positions 95-146 of the pre-proADM amino acid sequence and is a splice product thereof.

"Proadrenomedullin" ("Pro-ADM") refers to pre-proADM without the signal sequence (amino acids 1 to 21), i.e. to amino acid residues 22 to 285 of pre-proADM. "Midregional proadrenomedullin" ("MR-proADM") refers to the amino acids 42-95 of pre-proADM. The amino acid sequence of MR-proADM is given in SEQ ID NO: 2. It is also envisaged herein that a peptide and fragment thereof of pre-proADM or MR-proADM can be used for the herein described methods. For example, a peptide and fragment thereof can comprise amino acids 22-41 of pre-proADM (PAMP peptide) or amino acids 95-146 of pre-proADM (mature adrenomedullin). A C-terminal fragment of proADM (amino acids 153 to 185 of preproADM) is called adrenotensin. Fragments of proADM peptides or MR-proADM comprise for example 5 or more amino acids. Accordingly, the fragment of proADM may for example be selected from the group consisting of MR-proADM, PAMP, adrenotensin and mature adrenomedullin, preferably herein the fragment is MR-proADM.
Hence, fragments of proADM can in the context of the present invention preferably be selected from the group consisting of MR-proADM, PAMP, adrenotensin and mature adrenomedullin, most preferably herein the fragment is MR-proADM.

As mentioned herein in the context of proteins or peptides, such as proADM the term "fragment" refers to smaller proteins or peptides derivable from larger proteins or peptides, which hence comprise a partial sequence of the larger protein or peptide. Said fragments are derivable from the larger proteins or peptides by deletion of one or more of amino acids from the larger protein or peptide.

It is also envisaged herein that the level of a MR-proADM polypeptide is determined that has a sequence identity of at least 75%, for example, 75%, 80%, 85%, 90%, 91%, 92%, 93%, 94%, 95%, 96%, 97%, 98% or 99% sequence identity as shown in SEQ ID NO: 2, wherein the higher values of sequence identity are preferred. In accordance with the present invention, the terms "sequence identity", "homology" or "percent homology" or "identical" or "percent identity" or "percentage identity" in the context of two or more amino acid sequences refers to two or more sequences or subsequences that are the same, or that have a specified percentage of amino acids that are the same, when compared and aligned for maximum correspondence over the window of comparison (preferably over the full length), or over a designated region as measured using a sequence comparison algorithm as known in the art, or by manual alignment and visual inspection. Sequences having, for example, 70% to 90% or greater sequence identity may be considered to be substantially identical. Such a definition also applies to the complement of a test sequence. Preferably, the described identity exists over a region that is at least about 15 to about 20 amino acids in length, more preferably, over a region that is at least about 25 to about 45 amino acids in length, most preferably, over the full length. Those having skill in the art will know how to determine percent identity between/among sequences using, for example, algorithms such as those based on CLUSTALW computer program (Thompson Nucl. Acids Res. 2 (1994), 4673-4680) or FASTDB (Brutlag Comp. App. Biosci. 6 (1990), 237-245), as known in the art.

As used herein, the term "level of proadrenomedullin or a fragment thereof' refers to the quantity of the molecular entity of the marker proadrenomedullin or fragments thereof in a sample that is obtained from the subject. In other words, the concentration of the marker is determined in the sample. Hence, the term "level of the marker midregional proadrenomedullin (MR-proADM)" refers to the quantity of the molecular entity of the marker midregional proadrenomedullin (MR-proADM) in a sample that is obtained from the subject. As described above, it is also envisaged herein that a fragment of proadrenomedullin (proADM), preferably MR-proADM, can be detected and quantified. Also, fragments of MR-proADM can be detected and quantified. Suitable methods to determine the level of proADM or a fragment thereof (preferably MR-proADM) are described herein below in detail. Immunoassays in various formats such as for instance sandwich, enzyme-linked immunosorbent assay, luminescent immunoassay, rapid test formats, assays suitable for point-of-care testing and homogeneous assays such as, for example, the Kryptor system (BRAHMS/Thermo Fisher Scientific) can be employed. Moreover, mass spectrometry approaches can be used to detect and quantify proADM or a fragment thereof, preferably MR-proADM or a fragment thereof. The skilled person is aware of assays that are suitable to determine/quantify the herein described markers. Hence, in preferred aspects of the present invention, the level of proADM or a fragment thereof, preferably MR-proADM is determined in the sample, wherein said sample is a blood, serum or plasma sample. In most preferred aspects, the maker is determined in a plasma sample.
"Plasma" in the context of the present invention is the virtually cell-free supernatant of blood containing anticoagulant obtained after centrifugation. Exemplary anticoagulants include calcium ion binding compounds such as EDTA or citrate and thrombin inhibitors such as heparinates or hirudin. Cell-free plasma can be obtained by centrifugation of the anticoagulated blood (e.g. citrated, EDTA or heparinized blood), for example for at least 15 minutes at 2000 to 3000 g. "Serum" in the context of the present invention is the liquid fraction of whole blood that is collected after the blood is allowed to clot. When coagulated blood (clotted blood) is centrifuged serum can be obtained as supernatant.

In addition to the level of proADM or a fragment thereof, like MR-proADM, further parameters of the subject may be determined such as further markers, clinical scores and/or further clinical parameters. Hence, proADM or a fragment thereof, like MR-proADM, can be part of a marker panel.

As used herein, terms such as "marker", "surrogate", "prognostic marker", "factor" or "biomarker" or "biological marker" are used interchangeably and relate to measurable and quantifiable biological markers (e.g., specific enzyme concentration or a fragment thereof, specific hormone concentration or a fragment thereof, or presence of biological substances or a fragment thereof) which serve as indices for health- and physiology-related assessments, such as a disease/disorder/clinical condition risk. Furthermore, a biomarker is defined as a characteristic that is objectively measured and evaluated as an indicator of normal biological processes, pathogenic processes, or pharmacologic responses to a therapeutic intervention. A biomarker may be measured on a biological sample (as a blood, plasma, urine, or tissue test). As used herein, a parameter is a characteristic, feature, or measurable factor that can help in defining a particular system. A parameter is an important element for health- and physiology-related assessments, such as a disease/disorder/clinical condition risk. Furthermore, a parameter is defined as a characteristic that is objectively measured and evaluated as an indicator of normal biological processes, pathogenic processes, or pharmacologic responses to a therapeutic intervention.

An exemplary parameter can be selected from the group consisting of body mass index, weight, age, sex, IGS II, liquid intake, the simplified acute physiology score (SAPSII score), Acute Physiology and Chronic Health Evaluation II (APACHE II), World Federation of Neurosurgical Societies (WFNS) grading, Glasgow Coma Scale (GCS) and sequential organ failure assessment score (SOFA score), body mass index, weight, age, sex, liquid intake, white blood cell count, sodium concentration, potassium concentration, creatinine concentration, urine output, urine output within 24 hours, body temperature, blood pressure, dopamine, bilirubin, respiratory rate, partial pressure of oxygen, World Federation of Neurosurgical Societies (WFNS) grading, and Glasgow Coma Scale (GCS).

As used herein, the "sequential organ failure assessment score" or "SOFA score" is one score used to track a patient's status during the stay in an intensive care unit (ICU) (Vincent JL et al. The SOFA (Sepsis-related Organ Failure Assessment) score to describe organ dysfunction / failure. Intensive Care Med. 1996; 22:707-710). The SOFA score is a scoring system to determine the extent of a subject's organ function or rate of failure. The score is based on six different scores, one each for the respiratory, cardiovascular, hepatic, coagulation, renal and neurological systems. It assists doctors, nurses, and other members of the patient's health care team in estimating the risk of morbidity and mortality due to sepsis. The SOFA score can also be the so-called Quick SOFA score.

As used herein, the quick SOFA score (qSOFA) is a scoring system that indicates a patient's organ dysfunction or mortality risk. The score is based on three criteria: 1) an alteration in mental status, 2) a decrease in systolic blood pressure of less than 100 mm Hg, 3) a respiration rate greater than 22 breaths per minute. Patients with two or more of these conditions are at greater risk of having an organ dysfunction or to die.

The "Acute Physiology and Chronic Health Evaluation II" score (APACHE II) is a severity-of-disease classification system (Knaus et al., "APACHE II: a severity of disease classification system". In: Crit Care Med. 13(10), 1985, S. 818-829) one of several ICU scoring systems. It is applied within 24 hours of admission of a patient to an intensive care unit (ICU): an integer score from 0 to 71 is computed based on several measurements; higher scores correspond to more severe disease and a higher risk of death.

The "Simplified Acute Physiological (SAPS) II score" is a further severity of disease classification system (Le Gall, "A New Simplified Acute Physiology Score (SAPS II) Based on a European/North American Multicenter Study". JAMA. 1993; 270:2957-2963).

The at least one further marker and/or parameter of said subject can be selected from the group consisting of a level of lactate in said sample, a level of procalcitonin (PCT) in said sample, the sequential organ failure assessment score (SOFA score) of said subject, the simplified acute physiology score (SAPSII) of said subject, the Acute Physiology and Chronic Health Evaluation II (APACHE II) score of said subject and a level of any of the soluble fms-like tyrosine kinase-1 (sFlt-1), Histone H2A, Histone H2B, Histone H3, Histone H4, calcitonin, Endothelin-1 (ET-1), proArginine Vasopressin or Arginine Vasopressin (proAVP, AVP), Copeptin Atrial Natriuretic Peptide (ANP), Neutrophil Gelatinase-Associated Lipocalin (NGAL), Troponin, Brain Natriuretic Peptide (BNP), C-Reactive Protein (CRP), Pancreatic Stone Protein (PSP), Triggering Receptor Expressed on Myeloid Cells 1 (TREM1), Interleukin-6 (IL-6), Interleukin-1, Interleukin-24 (IL-24), Interleukin-22 (IL-22), Interleukin (IL-20) other ILs, Presepsin (sCD14-ST), Lipopolysaccharide Binding Protein (LBP), Alpha-1-Antitrypsin, Matrix Metalloproteinase 2 (MMP2), Metalloproteinase 8 (MMP8), Matrix Metalloproteinase 9 (MMP9), Matrix Metalloproteinase 7 (MMP7), Placental growth factor (P1GF), Chromogranin A, S100A protein, S100B protein and Tumor Necrosis Factor α (TNFα), Neopterin, atrial natriuretic peptide (ANP, pro-ANP), intercellular adhesion molecule 1 (ICAM-1), soluble intercellular adhesion molecule 1 (sICAM-1), CCL1/TCA3, CCL11, CCL12/MCP-5, CCL13/MCP-4, CCL14, CCL15, CCL16, CCL17/TARC, CCL18, CCL19, CCL2/MCP-1, CCL20, CCL21, CCL22/MDC, CCL23, CCL24, CCL25, CCL26, CCL27, CCL28, CCL3, CCL3L3, CCL4, CCL4L1/LAG-1, CCL5, CCL6, CCL7, CCL8, CCL9, CX3CL1, CXCL1, CXCL10, CXCL11, CXCL12, CXCL13, CXCL14, CXCL15, CXCL16, CXCL17, CXCL2/MIP-2, CXCL3, CXCL4, CXCL5, CXCL6, CXCL7/Ppbp, CXCL9, IL8/CXCL8, XCL1, XCL2, FAM19A1, FAM19A2, FAM19A3, FAM19A4, FAM19A5, CLCF1, CNTF, IL11, IL31, IL6, Leptin, LIF, OSM, IFNA1, IFNA10, IFNA13, IFNA14, IFNA2, IFNA4, IFNA7, IFNB1, IFNE, IFNG, IFNZ, IFNA8, IFNA5/IFNaG, IFNω/IFNW1, BAFF, 4-1BBL, TNFSF8, CD40LG, CD70, CD95L/CD178, EDA-A1, TNFSF14, LTA/TNFB, LTB, TNFα, TNFSF10, TNFSF11, TNFSF12, TNFSF13, TNFSF15, TNFSF4, IL18, IL18BP, ILIA, IL1B, IL1F10, IL1F3/IL1RA, IL1F5, IL1F6, IL1F7, IL1F8, IL1RL2, IL1F9, IL33 or a fragment thereof.

The method of the present invention, thus, in one aspect may further comprise the determination of the level of a further marker selected from the group consisting of procalcitonin (PCT), C-reactive protein (CRP), lactate and creatinine in a sample of said subject. The sample may be the same sample in which the level of proADM or a fragment thereof is determined or it can be a different sample.

As used herein, "lactate" refers to the maximal lactate concentration measured in the blood. Normally, the lactate concentration is assessed daily or even more often. The lactate concentration in the blood can for instance be determined by lactate oxidase spectrophotometric methods.

Because creatinine is a nonprotein end-product of creatine phosphate, which is used in skeletal muscle contraction, the daily production of creatine, and the following product, creatinine, depends on muscle mass, which fluctuates very little. Creatinine is excreted entirely by the kidneys, and therefore is directly related to renal function. When the kidneys are functioning normally, the serum creatinine level should remain constant and normal.

In addition to the level of proADM (or a fragment thereof) at least one clinical score of said subject selected from the group of SOFA score, SAPS II and APACHE II, is determined. Also, in addition the urine output, e.g. the urine output over 24 hours, of said subject can be determined.
However, surprisingly, the method of the present invention does not need to rely on further parameters and markers and in particular the cumbersome determination of urine output is not necessarily required.

In the context of the present invention, said level of proADM or the fragment thereof, preferably MR-proADM, is determined using a method selected from the group consisting of Luminescenceimmunoassay (LIA), radioimmunoassay (RIA), chemiluminescence- and fluorescence- immunoassay, enzyme immunoassay (EIA), Enzyme-linked immunoassay (ELISA), luminescence-based bead array, magnetic beads based array, protein microarray assay, rapid test formats, and rare cryptate assay. The assay can be performed in homogeneous phase or in heterogeneous phase as further outlined herein below.

The level of proADM or a fragment thereof, preferably MR-proADM and/or the level of further markers can be determined by an immunoassay. As used herein, an "assay" or a diagnostic assay can be of any type applied in the field of diagnostics. Preferred detection methods comprise immunoassays in various formats such as for instance radioimmunoassays, chemiluminescence- and fluorescence- immunoassays, Enzyme-linked immunoassays (ELISA), Luminex-based bead arrays, protein microarray assays, assays suitable for point-of-care testing and rapid test formats such as for instance immune-chromatographic strip tests. Such an assay may be based on the binding of an analyte to be detected to one or more capture probes with a certain affinity. As used herein, an immunoassay is a biochemical test that measures the presence or concentration of a macromolecule/polypeptide in a solution through the use of an antibody or immunoglobulin. According to the invention, the antibodies may be monoclonal as well as polyclonal antibodies. Thus, at least one antibody is a monoclonal or polyclonal antibody. The method according to the present invention is particularly preferred, wherein the midregional partial peptide spanning amino acids 42-95 of pre-proADM or amino acids as given in SEQ ID NO: 2 is employed for the determination of MR-proADM or partial peptides thereof in a sample. In certain aspects, the level of the marker is determined by high performance liquid chromatography (HPLC). In certain aspects, the HPLC can be coupled to an immunoassay. In certain aspects of the present invention, proADM or a fragment thereof, preferably MR-proADM or a fragment thereof. In this sandwich immunoassay, two antibodies are applied for, e.g., one marker such as proADM or a fragment thereof, preferably MR-proADM, in a sample. In particular, this is preferred, if proADM or a fragment thereof, preferably MR-proADM or a fragment thereof are determined by the use of two antibodies, which specifically bind to different partial sequences of proADM or a fragment thereof, preferably MR-proADM or a fragment thereof.
In a preferred aspect of the *in vitro* method according the invention, one of the antibodies is labeled and the second one is bound to or may be bound selectively to a solid phase.
In a particularly preferred aspect of the assay, one of the antibodies is labeled while the other is either bound to a solid phase or can be bound selectively to a solid phase. In a preferred embodiment the method is executed as heterogeneous sandwich immunoassay, wherein one of the antibodies is immobilized on an arbitrarily chosen solid phase, for example, the walls of coated test tubes (e.g. polystyrol test tubes; coated tubes; CT) or microtiter plates, for example composed of polystyrol, or to particles, such as for instance magnetic particles, whereby the other antibody has a group resembling a detectable label or enabling for selective attachment to a label, and which serves the detection of the formed sandwich structures. A temporarily delayed or subsequent immobilization using suitable solid phases is also possible.

The method according to the present invention can furthermore be embodied as a homogeneous method, wherein the sandwich complexes formed by the antibody/antibodies and the marker, e.g., proADM or a fragment thereof, preferably MR-proADM or a fragment thereof, which is to be detected remains suspended in the liquid phase. In this case it is preferred, that when two antibodies are used, both antibodies are labeled with parts of a detection system, which leads to generation of a signal or triggering of a signal if both antibodies are integrated into a single sandwich. Such techniques are to be embodied in particular as fluorescence enhancing or fluorescence quenching detection methods. A particularly preferred aspect relates to the use of detection reagents which are to be used pair-wise, such as for example the ones which are described in US 4 882 733 A, EP-B1 0 180 492 or EP-B1 0 539 477 and the prior art cited therein. In this way, measurements in which only reaction products comprising both labeling components in a single immune-complex directly in the reaction mixture are detected, become possible. For example, such technologies are offered under the brand names TRACE® (Time Resolved Amplified Cryptate Emission) or KRYPTOR®, implementing the teachings of the above-cited applications. Therefore, in particular preferred aspects, a diagnostic device is used to carry out the herein provided method. For example, the level of proADM or a fragment thereof, preferably MR-proADM and/or the level of any further marker of the herein provided method is determined. In particular preferred aspects, the diagnostic device is KRYPTOR®.

In a preferred aspect, both the first and the second antibody are dispersed in the liquid reaction medium, whereby a first labeling component which is part of a labeling system based on fluorescence- or chemoluminescence quenching or enhancement is bound to the first antibody, and whereby the second labeling component of this labeling system is bound to the second antibody, such that after binding of both antibodies to proADM or a fragment thereof, preferably MR-proADM, a detectable signal is generated which enables for a detection of the sandwich complexes formed in the measuring solution. One aspect of this alternative comprises the labeling system such as rare earth cryptates or chelates in combination with a fluorescence- or cheminoluminescence-dye. In a particular preferred aspect, the labeling system comprises a rare earth cryptate in combination with a fluorescence or chemiluminescence dye, in particular, of the cyanine type. In a further preferred aspect, the detection is carried out with a competitive immunoassay. In a preferred aspect, a radioimmunoassay is used. It also envisaged herein that the level of the marker can be, for example, determined by mass spectrometric methods or by a high performance liquid chromatography (HPLC) method, which can be coupled to an immunoassay, or a mass-spectrometric based approach. The skilled person understands that any available assay can be used as long as the level of the marker can be reliably determined.

In one aspect, the method is an immunoassay comprising the steps of:
a) contacting the sample with
   (i) a first antibody or an antigen-binding fragment or derivative thereof specific for a first epitope of proADM or the fragment thereof, preferably MR-proADM, and
   (ii) a second antibody or an antigen-binding fragment or derivative thereof specific for a second epitope of proADM or the fragment thereof, preferably MR-proADM; and
b) detecting the binding of the first and second antibodies or antigen-binding fragments or derivates thereof to proADM or the fragment thereof, preferably MR-proADM.
Typically, in this context one of the antibodies is labeled and the other antibody is bound to a solid phase or can be bound selectively to a solid phase. For example, the first antibody and the second antibody are present dispersed in a liquid reaction mixture, and wherein a first labelling component which is part of a labelling system based on fluorescence or chemiluminescence extinction or amplification is bound to the first antibody, and a second labelling component of said labelling system is bound to the second antibody so that, after binding of both antibodies to proADM or the fragment thereof, preferably MR-proADM, a measurable signal which permits detection of the resulting sandwich complexes in the measuring solution is generated.
As indicated herein above, labelling systems may comprise a rare earth cryptate or chelate in combination with a fluorescent or chemiluminescent dye, in particular of the cyanine type.

In principle, all labeling techniques which can be applied in assays of said type can be used, such as labeling with radioisotopes, enzymes, fluorescence-, chemoluminescence- or bioluminescence labels and directly optically detectable color labels, such as gold atoms and dye particles, which are used in particular in Point-of-Care (POC) or rapid tests. In the case of heterogeneous sandwich immunoassays, both antibodies may exhibit parts of the detection system according to the type described herein in the context of homogenous assays.

As used herein, the "therapy" or "treatment" is particularly renal replacement therapy. As used herein, "renal replacement therapy" or "RRT" is a therapy that is employed to replace or support the normal blood-filtering function of the kidney(s). Particularly, renal replacement therapy may be continuous renal replacement therapy and/or intermittent renal replacement therapy. As used herein, intermittent renal replacement therapy relates to a process of blood purification/solute clearance based e.g. on diffusion across the membrane driven by a concentration gradient between the blood and dialysate. As used herein, continuous renal replacement therapy is any renal replacement therapy that is intended to be applied for 24 h per day, i.e. continuously. Renal replacement therapy may refer to dialysis (e.g. hemodialysis or peritoneal dialysis), hemofiltration, and hemodiafiltration. Such techniques are various ways of diverting the blood into a machine, cleaning it, and then returning it to the body. Renal replacement therapy may also refer to kidney transplantation, which is the ultimate form of replacement in that the old kidney is replaced by a donor kidney. Renal replacement therapy may also be selected from the group consisting of dialysis, hemofiltration, hemodiafiltration and kidney transplantation. The hemodialysis, hemofiltration, and hemodiafiltration may be continuous or intermittent and can use an arteriovenous route (in which blood leaves from an artery and returns via a vein) or a venovenous route (in which blood leaves from a vein and returns via a vein). This results in various types of RRT. For example, the renal replacement therapy may be selected from the group of continuous renal replacement therapy (CRRT), continuous hemodialysis (CHD), continuous arteriovenous hemodialysis (CAVHD), continuous venovenous hemodialysis (CVVHD), continuous hemofiltration (CHF), continuous arteriovenous hemofiltration (CAVH or CAVHF), continuous venovenous hemofiltration (CVVH or CVVHF), continuous hemodiafiltration (CHDF), continuous arteriovenous hemodiafiltration (CAVHDF), continuous venovenous hemodiafiltration (CVVHDF), intermittent renal replacement therapy (IRRT), intermittent hemodialysis (IHD), intermittent venovenous hemodialysis (IVVHD), intermittent hemofiltration (IHF), intermittent venovenous hemofiltration (IVVH or IVVHF), intermittent hemodiafiltration (IHDF) and intermittent venovenous hemodiafiltration (IVVDF).

As indicated herein above, the sensitivity and specificity of a diagnostic and/or prognostic test such as the method of the present invention depends on more than just the analytical "quality" of the test, they also depend on the definition of what constitutes an abnormal result. In practice, Receiver Operating Characteristic curves (ROC curves), are typically calculated by plotting the value of a variable versus its relative frequency in "normal" (i.e. apparently healthy individuals not having a prenatal disorder or condition) and "disease" populations. For any particular marker (like MR-proADM), a distribution of marker levels for subjects with and without a disease/condition will likely overlap. Under such conditions, a test does not absolutely distinguish normal from disease with 100% accuracy, and the area of overlap might indicate where the test cannot distinguish normal from disease. A threshold is selected, below which the test is considered to be abnormal and above which the test is considered to be normal or below or above which the test indicates a specific condition. The area under the ROC curve is a measure of the probability that the perceived measurement will allow correct identification of a condition. ROC curves can be used even when test results do not necessarily give an accurate number. As long as one can rank results, one can create a ROC curve. For example, results of a test on "disease" samples might be ranked according to degree (e.g. 1=low, 2=normal, and 3=high). This ranking can be correlated to results in the "normal" population, and a ROC curve created. These methods are well known in the art; see, e.g., Hanley et al. 1982. Radiology 143: 29-36. Preferably, a threshold is selected to provide a ROC curve area of greater than about 0.5, more preferably greater than about 0.7, still more preferably greater than about 0.8, even more preferably greater than about 0.85, and most preferably greater than about 0.9. The term "about" in this context refers to +/- 5% of a given measurement.
The horizontal axis of the ROC curve represents (1-specificity), which increases with the rate of false positives. The vertical axis of the curve represents sensitivity, which increases with the rate of true positives. Thus, for a particular cut-off selected, the value of (1-specificity) may be determined, and a corresponding sensitivity may be obtained. The area under the ROC curve is a measure of the probability that the measured marker level will allow correct identification of a disease or condition. Thus, the area under the ROC curve (AUC) can be used to determine the effectiveness of the test.

In other embodiments, a positive likelihood ratio, negative likelihood ratio, odds ratio, or hazard ratio is used as a measure of a test's ability to predict risk or diagnose a disorder or condition ("diseased group"). In the case of a positive likelihood ratio, a value of 1 indicates that a positive result is equally likely among subjects in both the "diseased" and "control" groups; a value greater than 1 indicates that a positive result is more likely in the diseased group; and a value less than 1 indicates that a positive result is more likely in the control group. In the case of a negative likelihood ratio, a value of 1 indicates that a negative result is equally likely among subjects in both the "diseased" and "control" groups; a value greater than 1 indicates that a negative result is more likely in the test group; and a value less than 1 indicates that a negative result is more likely in the control group.

In the case of an odds ratio, a value of 1 indicates that a positive result is equally likely among subjects in both the "diseased" and "control" groups; a value greater than 1 indicates that a positive result is more likely in the diseased group; and a value less than 1 indicates that a positive result is more likely in the control group.

In the case of a hazard ratio, a value of 1 indicates that the relative risk of an endpoint (e.g., death) is equal in both the "diseased" and "control" groups; a value greater than 1 indicates that the risk is greater in the diseased group; and a value less than 1 indicates that the risk is greater in the control group. "Diseased" and "control" groups herein are representative for two groups of different condition (e.g. "need of RRT" and "no need of RRT").

The skilled artisan will understand that associating a diagnostic or prognostic indicator, with a diagnosis or with a prognostic risk of a future clinical outcome is a statistical analysis. For example, a marker level of lower than X may signal that a patient is more likely to suffer from an adverse outcome than patients with a level more than or equal to X, as determined by a level of statistical significance. Additionally, a change in marker concentration from baseline levels may be reflective of patient prognosis, and the degree of change in marker level may be related to the severity of adverse events. Statistical significance is often determined by comparing two or more populations, and determining a confidence interval and/or a p value; see, e.g., Dowdy and Wearden, Statistics for Research, John Wiley & Sons, New York, 1983. Preferred confidence intervals of the invention are 90%, 95%, 97.5%, 98%, 99%, 99.5%, 99.9% and 99.99%, while preferred p values are 0.1, 0.05, 0.025, 0.02, 0.01, 0.005, 0.001, and 0.0001.

The present invention also relates to the use of a kit for assessing whether a critically ill subject is in need of a renal replacement therapy, wherein the kit comprises one or more detection reagents for determining the level of proADM or said fragment thereof, preferably MR-proADM, in a sample of said subject, optionally wherein said detection reagents comprise at least two antibodies, wherein one of the antibodies is labelled and the other antibody is bound to a solid phase or can be bound selectively to a solid phase.
In the context of this use, a first and a second antibody may be present dispersed in a liquid reaction mixture, and wherein a first labelling component that is part of a labelling system based on fluorescence or chemiluminescence extinction or amplification is bound to the first antibody, and a second labelling component of said labelling system is bound to the second antibody so that, after binding of both antibodies to proADM or said fragment thereof, preferably MR-proADM, a measurable signal which permits detection of the resulting sandwich complexes in the measuring solution is generated, optionally wherein said labelling system comprises rare earth cryptates or chelates in combination with a fluorescent or chemiluminescent dye, in particular of the cyanine type.

The present invention also pertains to a method for treating a critically ill subject with a renal replacement therapy, comprising assessing whether said critically ill subject is in need of said renal replacement therapy, wherein the method comprises determining in a sample obtained from said subject the level of proadrenomedullin (proADM) or a fragment thereof, preferably MR-proADM, wherein said level of proADM or the fragment thereof is indicative of a need of said subject to receive a renal replacement therapy.

In one embodiment of the method described herein, the method additionally comprises comparing the determined level of proADM or the fragment thereof to a reference level, threshold value and/or a population average corresponding to proADM or fragments thereof in patients who have been diagnosed as being critically ill and are under medical treatment, wherein said comparing is carried out in a computer processor using computer executable code.

The methods of the present invention may in part be computer-implemented. For example, the step of comparing the detected level of a marker, e.g. the proADM or the fragment thereof, with a reference level can be performed in a computer system. In the computer-system, the determined level of the marker(s) can be combined with other marker levels and/or parameters of the subject in order to calculate a score, which is indicative for the diagnosis, prognosis, risk assessment and/or risk stratification. For example, the determined values may be entered (either manually by a health professional or automatically from the device(s) in which the respective marker level(s) has/have been determined) into the computer-system. The computer-system can be directly at the point-of-care (e.g. primary care, ICU or ED) or it can be at a remote location connected via a computer network (e.g. via the internet, or specialized medical cloud-systems, optionally combinable with other IT-systems or platforms such as hospital information systems (HIS)). Typically, the computer-system will store the values (e.g. marker level or parameters such as age, blood pressure, weight, sex, etc. or clinical scoring systems such as SOFA, qSOFA, BMI etc.) on a computer-readable medium and calculate the score based-on pre-defined and/or pre-stored reference levels or reference values. The resulting score will be displayed and/or printed for the user (typically a health professional such as a physician). Alternatively or in addition, the associated prognosis, diagnosis, assessment, treatment guidance, patient management guidance or stratification will be displayed and/or printed for the user (typically a health professional such as a physician).

In one embodiment of the invention, a software system can be employed, in which a machine learning algorithm is evident, preferably to identify hospitalized patients at risk for sepsis, severe sepsis and septic shock using data from electronic health records (EHRs). A machine learning approach can be trained on a random forest classifier using EHR data (such as labs, biomarker expression, vitals, and demographics) from patients. Machine learning is a type of artificial intelligence that provides computers with the ability to learn complex patterns in data without being explicitly programmed, unlike simpler rule-based systems. Earlier studies have used electronic health record data to trigger alerts to detect clinical deterioration in general. In one embodiment of the invention the processing of proADM levels may be incorporated into appropriate software for comparison to existing data sets, for example proADM levels may also be processed in machine learning software to assist in diagnosing or prognosing the occurrence of an adverse event or the need of the subject for renal replacement therapy.

All references cited herein are fully incorporated by reference.

### Sequences

SEQ ID NO:1: amino acid sequence of pre-pro-ADM:

1 MKLVSVALMY LGSLAFLGAD TARLDVASEF RKKWNKWALS RGKRELRMSS

51 SYPTGLADVK AGPAQTLIRP QDMKGASRSP EDSSPDAARI RVKRYRQSMN

101 NFQGLRSFGC RFGTCTVQKL AHQIYQFTDK DKDNVAPRSK ISPQGYGRRR

151 RRSLPEAGPG RTLVSSKPQA HGAPAPPSGS APHFL

SEQ ID NO:2: amino acid sequence of MR-pro-ADM (AS 45-92 of pre-pro-ADM):

### 1 ELRMSSSYPT GLADVKAGPA QTLIRPQDMK GASRSPEDSS PDAARIRV

The following non-binding Example illustrates the invention.

### Example

The Example has been performed by detecting MR-proADM. However, as outlined herein above, the invention can also be performed by detecting proADM or another peptide fragment thereof.

### Study design

A total of 1089 intensive care unit (ICU) patients were enrolled in the study, with 142 (13.0%) patients suffering from severe sepsis, and 947 (87.0%) patients suffering from septic shock. The 28 day mortality rate was 26.9%, with a hospital mortality rate of 33.4%. Within the first 28 days of ICU therapy, 321 (29.8%) patients required renal replacement therapy (RRT), with the vast majority of patients (N = 296; 92.2%) requiring RRT for the first time within the first 7 days of treatment
- Baseline: 178(16.6%)
- Day 1: 59 (6.7%)
- Day 2: 25 (3.2%)
- Day 3: 11 (1.6%)

An increasing trend in 28 or 90 day mortality based on the time when RRT was initiated could be found:
- Baseline 28 and 90 day mortality rates: 52.2% and 63.2%;
- Day 1 28 and 90 day mortality rates: 57.6% and 72.9%;
- Day 2 28 and 90 day mortality rates: 44.0% and 56.0%;
- Day 3 28 and 90 day mortality rates: 63.6% and 72.7%.

Biomarkers and clinical severity scores were assessed for their performance on baseline for (i) identifying patients requiring RRT at baseline, (ii) predicting RRT requirement on day 1, (iii) predicting RRT requirement on day 2, and (iii) predicting RRT requirement on day 3.

### Biomarker Measurements

PCT was measured in serum samples on devices with a measuring range of 0.02 - 5000 ng/ml, and a functional assay sensitivity and lower detection limit of at least 0.06 ng/ml and 0.02 ng/ml (BRAHMS PCT assay for Kryptor®, Thermo Fisher Scientific, Germany), respectively. Additional blood samples from all patients were collected stored at -80 °C. MR-proADM plasma concentrations were measured retrospectively (Kryptor®, Thermo Fisher Scientific, Germany) with a limit of detection of 0.05 nmol/L. Clinical severity scores including the Sequential Organ Failure Assessment (SOFA), Acute Physiological and Chronic Health Evaluation (APACHE) II and Simplified Acute Physiological (SAPS) II score were taken upon study enrolment. Creatinine was determined in the urine using a standard commercially available assay kit. Plasma c-reactive protein (CRP) was determined using a standard commercially available assay kit. Plasma lactate was determined using a standard commercially available assay kit.

### Results

Results are summarized in Table 1.

**Table 1: Summary of results (AUC values)**

| Biomarker/Clinical score | RRT requirement on admission | Baseline values to day 1 requirement | Baseline values to day 2 requirement | Baseline values to day 3 requirement |
|---|---|---|---|---|
| MR-proADM | 0.823 | 0.831 | 0.803 | 0.795 |
| PCT | 0.670 | 0.683 | 0.659 | 0.598 |
| CRP | 0.554 | 0.507 | 0.588 | 0.658 |
| Lactate | 0.722 | 0.732 | 0.595 | 0.639 |
| Creatinine max | 0.798 | 0.768 | 0.777 | 0.646 |
| Creatinine min | 0.779 | 0.775 | 0.784 | 0.639 |
| Urine output (24 hrs) | 0.893 | 0.786 | 0.791 | 0.623 |
| SOFA | 0.781 | 0.717 | 0.656 | 0.600 |
| SAPS II | 0.733 | 0.650 | 0.623 | 0.630 |
| APACHE II | 0.771 | 0.665 | 0.589 | 0.717 |

MR-proADM had the greatest predictive value compared to all other biomarkers, score or laboratory values. Only urine output over 24 hours could identify patients more accurately at baseline than MR-proADM, however this variable requires constant measurement over a 24 hour period as opposed to a single biomarker measurement on admission. Furthermore, whilst values remained high for day 1 and day 2 prediction, they were lower than for MR-proADM, especially when comparing day 3 predictions.

In a second step, baseline and day 1 PCT and MR-proADM kinetics were investigated (see Table 2). Patients were firstly split into whether they had increasing or decreasing PCT values over this 24 hour period, and then further subgroups were investigated using MR-proADM concentrations (baseline: low severity ≤2.7 nmol/L; Intermediate severity between >2.7 nmol/L and ≤10.9 nmol/L; high severity >10.9 nmol/L; day 1: low severity ≤2.8 nmol/L; Intermediate severity between >2.8 nmol/L and ≤9.5 nmol/L; high severity >9.5 nmol/L).
A total of 165 patients had decreasing PCT concentrations from baseline to day 1 and a constantly low MR-proADM concentration. Of these patients, only 4 (2.4%) patients required RRT. Of the remaining 161 patients, there was no further requirement for RRT up to an including day 7. In contrast, 51 (7.9%) patients were found to have decreasing PCT concentrations yet a constantly high MR-proADM concentration. Here, 23 (45.1 %) patients required RRT at baseline. Of the remaining 23 patients, 12 (52.2%) required RRT at some point over the subsequent 7 days.
Similar results could be found when PCT values were increasing from baseline to day 1. In this case, 66 (19.2%) of patients had an increasing PCT concentration but a constantly low MR-proADM severity value. No patients required RRT at baseline, but of these patients, 3 (4.5%) required RRT at some point over the subsequent 7 days of ICU therapy. In contrast, 53 (15.4%) patients had increasing PCT concentrations and a constantly high MR-proADM severity value. 22 (41.5%) of these patients required RRT immediately at baseline, whereas of the 31 patients who were not put on RRT immediately, a further 23 (74.2%) required RRT over the course of the next 7 days.

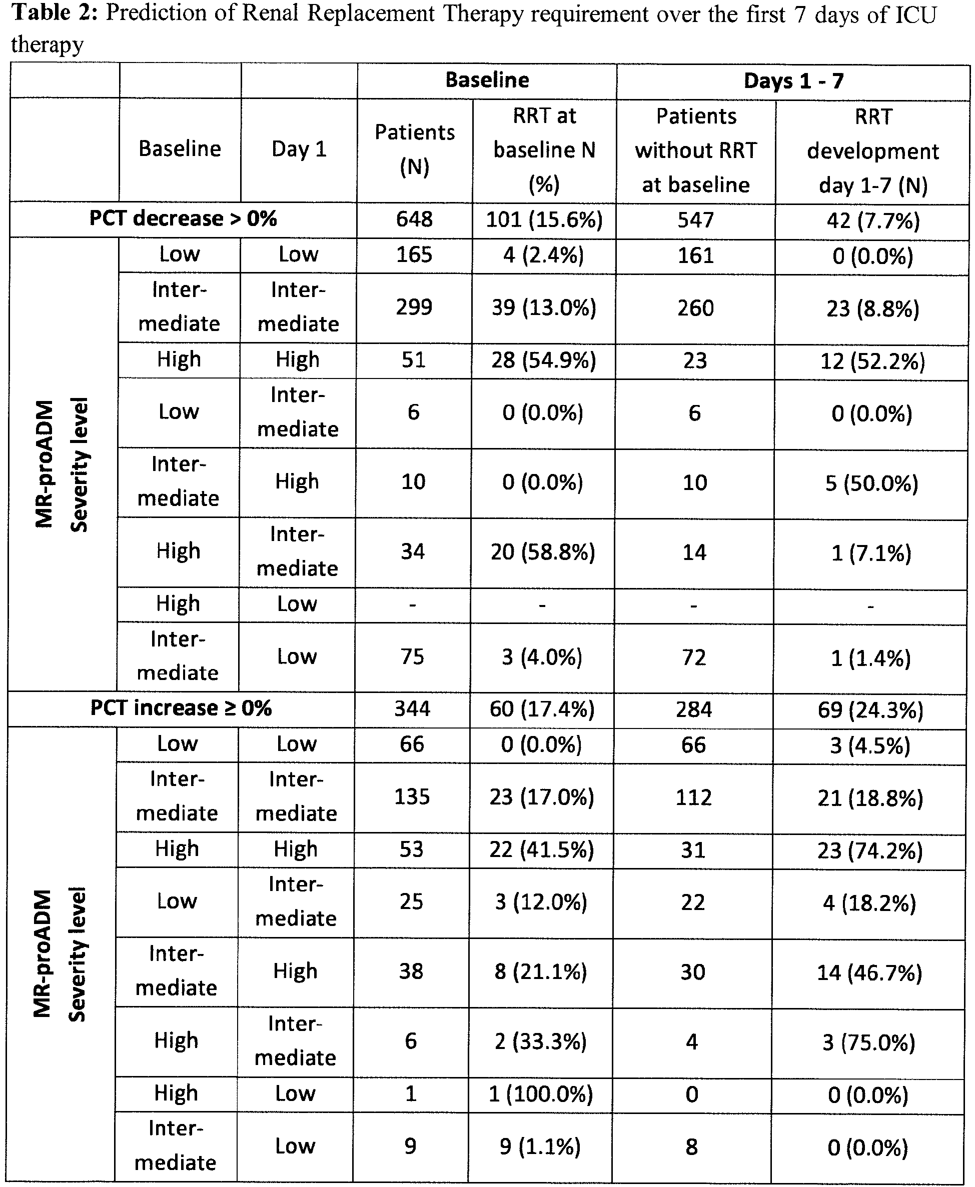

## Claims

1. A method for the therapy control, therapy guidance, monitoring, risk assessment, and/or risk stratification of a critically ill subject, wherein said method comprises determining the level of proadrenomedullin (proADM) or a fragment thereof, preferably MR-proADM, in a sample of said subject, wherein said level of proADM or a fragment thereof, preferably MR-proADM, is indicative of a need of said subject to receive a therapy, particularly renal replacement therapy.

2. A method for assessing whether a critically ill subject is in need of a renal replacement therapy, wherein the method comprises determining in a sample obtained from said subject the level of proadrenomedullin (proADM) or a fragment thereof, preferably MR-proADM, wherein said level of proADM or the fragment thereof is indicative of a need of said subject to receive a renal replacement therapy.

3. The method of claim 1 or 2, wherein said level of proADM or the fragment thereof is compared to a reference level, and a need of said subject to receive said therapy is identified based on the comparison of the level of proADM or the fragment thereof with the reference level, wherein
(a) a level of proADM or said fragment thereof, preferably MR-proADM, above said reference level in the sample is indicative for said subject's need of said therapy, particularly renal replacement therapy; and
(b) a level of proADM or said fragment thereof, preferably MR-proADM, equal or below said reference level is indicative for termination of a renal replacement therapy if said subject is receiving a renal replacement therapy.

4. The method of claim 3(a), wherein said reference level is a pre-determined level of a population of healthy subjects who are not in need of renal replacement therapy, or wherein said reference level is a pre-determined level of a population of critically ill subjects who are not in need of renal replacement therapy.

5. The method of claim 4, wherein said reference level is selected in the range of from 1 nmol/L to 12 nmol/L, preferably in the range of from 2 nmol/L to 11 nmol/L, more preferably in the range of from 2.5 nmol/L to 11 nmol/L, even more preferably between in the range of from 9 nmol/L to 11 nmol/L.

6. The method of claim 5, wherein said reference level is selected from the group consisting of 2.7 nmol/L, 2.8 nmol/L, 9.5 nmol/L and 10.9 nmol/L.

7. The method of claim 3(b), wherein a level of proadrenomedullin (proADM) or the fragment thereof equal or below 2.0 nmol/L, preferably equal or below 2.5 nmol/L, most preferably equal or below 2.7 nmol/L, is indicative for termination of said renal replacement therapy.

8. The method according to any one of the preceding claims, wherein said critically ill subject is a subject undergoing intensive care or is a patient in an intensive care unit (ICU).

9. The method according to any one of the preceding claims, wherein the critically ill subject suffers from sepsis or septic shock or is suspected to suffer from sepsis or septic shock.

10. The method of any one of the preceding claims, wherein said sample is a sample of a bodily fluid, preferably blood, plasma or serum, more preferably plasma or serum.

11. The method according to any one of the preceding claims, wherein the fragment of proADM is selected from the group consisting of MR-proADM, PAMP, adrenotensin and mature adrenomedullin, preferably the fragment is MR-proADM.

12. The method of any one of the preceding claims, wherein said method further comprises
(a) the determination of the level of a further marker selected from the group consisting of Procalcitonin (PCT), C-reactive Protein (CRP), Lactate, Endothelin-1, proArginine Vasopressin, Copeptin, Neutrophil Gelatinase-Associated Lipocalin (NGAL), Interleukin-6 and creatinine in a sample of said subject; and/or
(b) the determination of the urine output, preferably the urine output over 24 hours, of said subject; and/or
(c) the determination of at least one clinical score of said subject selected from the group of SOFA score, SAPS II and APACHE II.

13. The method of any one of the preceding claims, wherein said level of proADM or the fragment thereof, preferably MR-proADM, is determined using a method selected from the group consisting of Luminescenceimmunoassay (LIA), radioimmunoassay (RIA), chemiluminescence- and fluorescence- immunoassay, enzyme immunoassay (EIA), Enzyme-linked immunoassay (ELISA), luminescence-based bead array, magnetic beads based array, protein microarray assay, rapid test formats, and rare cryptate assay.

14. The method of any one of the preceding claims, wherein the method is an immunoassay comprising the steps of:
a) contacting the sample with
(i) a first antibody or an antigen-binding fragment or derivative thereof specific for a first epitope of proADM or the fragment thereof, preferably MR-proADM, and
(ii) a second antibody or an antigen-binding fragment or derivative thereof specific for a second epitope of proADM or the fragment thereof, preferably MR-proADM; and
b) detecting the binding of the first and second antibodies or antigen-binding fragments or derivates thereof to proADM or the fragment thereof, preferably MR-proADM;
optionally wherein the first antibody and the second antibody are present dispersed in a liquid reaction mixture, and wherein a first labelling component which is part of a labelling system based on fluorescence or chemiluminescence extinction or amplification is bound to the first antibody, and a second labelling component of said labelling system is bound to the second antibody so that, after binding of both antibodies to proADM or the fragment thereof, preferably MR-proADM, a measurable signal which permits detection of the resulting sandwich complexes in the measuring solution is generated.

15. Use of a kit for assessing whether a critically ill subject is in need of a renal replacement therapy, wherein the kit comprises one or more detection reagents for determining the level of proADM or said fragment thereof, preferably MR-proADM, in a sample of said subject, optionally wherein said detection reagents comprise at least two antibodies, wherein one of the antibodies is labelled and the other antibody is bound to a solid phase or can be bound selectively to a solid phase.
